# EUROPÄISCHE PATENTSCHRIFT

(11) **EP 2 392 374 B1**
(45) Veröffentlichungstag und Bekanntmachung des Hinweises auf die Patenterteilung: **08.06.2016**
(21) Anmeldenummer: 11170495.3
(22) Anmeldetag: 11.11.2004
(51) Int. Cl.: A61M 16/06

(54) **Kopfbandeinrichtung für eine Atemmaske**
Headband device for a breathing mask
Dispositif de serre-tête pour masque respiratoire

(30) Priorität: 11.11.2003 DE 10352608; 11.11.2003 DE 10352607
(43) Veröffentlichungstag der Anmeldung: 07.12.2011
(62) Teilanmeldung aus: 04818394.1
(73) Patentinhaber: ResMed R&D Germany GmbH, 82152 Martinsried (DE)
(72) Erfinder: Lang, Bernd, 82166 Gräfelfing (DE); Biener, Achim, 85445 Aufkirchen (DE); Bechtel, Martin, 21423 Winsen/Luhe (DE); Vögele, Harald, 82131 Gauting (DE); Stauffenberg, Graf Caspar, 82131 Gauting (DE)
(74) Vertreter: Vossius & Partner Patentanwälte Rechtsanwälte mbB

(56) Entgegenhaltungen:
- WO-A1-00/50122
- DE-A1- 19 807 961
- US-A- 3 013 556
- US-A- 4 367 735
- US-A- 5 265 595

## Beschreibung

Die Erfindung betrifft eine Applikationseinrichtung für eine Atemmaske mit einer Kopfbandeinrichtung zur Applikation der Atemmaske im Gesichtsbereich eines Anwenders.

Atemmasken finden insbesondere im medizinischen bzw. therapeutischen Bereich zur Behandlung schlafbezogener Atmungsstörungen Anwendung. Durch derartige Atemmasken ist es möglich, einem Anwender ein atembares Gas, insbesondere gefilterte Umgebungsluft, auf einem Druckpegel zuzuführen, der über dem Umgebungsdruck liegt. Durch die derart bei erhöhtem Druck vorgenommene Atemgasversorgung wird es möglich, im Bereich der oberen Atemwege eine pneumatische Schienung zu erreichen und hierdurch etwaigen Obstruktionen in diesem Atemwegsbereich vorzubeugen. Die derart zur Behandlung schlafbezogener Atmungsstörungen verwendeten Atemmasken werden vom Anwender über die gesamte Ruhe- bzw. Schlafphase getragen. Diese Atemmasken werden üblicherweise über eine Kopfbandanordnung am Kopf des Anwenders fixiert. Diese Kopfbandanordnungen können einen oberen Kopfbandabschnitt sowie einen unteren Kopfbandabschnitt umfassen, wobei über den oberen Kopfbandabschnitt entsprechende Haltekräfte auf eine Stirnauflageeinrichtung der Atemmaske aufgebracht werden können. Durch den unteren Kopfbandabschnitt kann die Atemmaske gegen den Umgebungsbereich der Nase, den Nasenrückenbereich sowie den Oberlippenbereich gedrängt werden.

Dokument US 5,265,595 zeigt eine Atemmaske, die mittels einer flexiblen Kappe und anpassbaren Bändern befestigt wird. Dokument WO 00/50122 offenbart eine becherartige Atemmaske, die mittels Schnüren und einem Band positioniert wird. Dokument US 4,367,735 lehrt eine Nasenbrille, die mittels Schaumstoffbändern und einer Kappe gehalten wird. Dokument US 3,013,556 offenbart eine Atemmaske, die mittels Schlaufen und einem Kopfband befestigt wird.

Die Erfindung betrifft eine Applikationsvorrichtung für eine Atemmaske, insbesondere für Atemmaske zur Behandlung schlafbezogener Atmungsstörungen.

Der Erfindung liegt die Aufgabe zugrunde, eine Applikationsvorrichtung für eine Atemmaske zu schaffen, die eine zuverlässige Fixierung einer Atemmaske ermöglicht und sich durch einen hohen Anwendungskomfort auszeichnet.

Diese Aufgabe wird erfindungsgemäß gelöst durch eine Applikationsvorrichtung für eine Atemmaske gemäß Anspruch 1.

Dadurch wird es auf vorteilhafte Weise möglich, eine Kopfbandanordnung zu schaffen, durch welche ein, auf die individuelle Kopfform des Maskenanwenders vorteilhaft abgestimmter Verlauf der sich zur Atemmaske hin erstreckenden Kopfbandabschnitte gewährleistet ist. Weiterhin wird es auch in besonders vorteilhafter Weise möglich, die über die Kopfbandabschnitte aufgebrachten Zugkräfte unter einer geringen Flächenpressung in den Hinterkopfbereich des Maskenanwenders einzuleiten.

Vorzugsweise ist die Kopfbandanordnung derart gestaltet, dass die Stützstruktur auf Ohrhöhe, oder im Bereich des Halswirbelauslaufs auf dem Hinterkopfbereich des Anwenders aufsitzt.

Gemäß einer besonders bevorzugten Ausführungsform der Erfindung ist die Stützstruktur aus einem, nach Erwärmung auf eine Temperatur von vorzugsweise wenigstens 30°C plastisch verformbaren Material gefertigt. Die Erwärmung der Stützstruktur kann beispielsweise in einem Wasserbad oder durch Heißluft erfolgen.

Die Stützstruktur ist vorzugsweise aus einem thermo-plastischen Kunststoffmaterial, gefertigt. Die Stützstruktur kann so ausgebildet sein, dass diese eine Versteifungslage bildet, die beispielsweise in noch nicht vollständig ausgehärtetem Zustand an die Hinterkopfwölbung des Anwenders anpassbar ist. Die Stützstruktur kann auf ihrer, in Applikationsposition einem Anwender, zugewandten Seite, mit einer Polsterung versehen sein. Diese Polsterung kann aus einem Schaumstoff- und/oder Vliesmaterial gebildet sein.

Insbesondere bei einer Ausgestaltung der Stützstruktur als gewölbtes Plattenelement ist diese vorzugsweise mit Durchbrechungen versehen. Hierdurch wird die Dampfdurchlässigkeit der Stützstruktur erhöht und einer Überfeuchtung der Kopfbandeinrichtung vorgebeugt. Es ist möglich, die Stützstruktur derart auszubilden, dass diese lösbar mit der Kopfbandeinrichtung gekoppelt ist. Dadurch wird es auf vorteilhafte Weise möglich, die Kopfbandeinrichtung von der Stützstruktur zu trennen und die Kopfbandeinrichtung separat zu reinigen.

Gemäß einer besonders bevorzugten Ausführungsform der Erfindung ist die Stützstruktur derart ausgebildet, dass diese Armabschnitte aufweist, die sich von einem Hauptauflageflächenabschnitt ausgehend entlang der Kopfbandabschnitte erstrecken.

Die Stützstruktur kann derart ausgebildet sein, dass diese einen unteren Randabschnitt mit zwei unteren Schenkeln aufweist, die sich in Applikationsposition zum Wangenknochen bzw. in einen unter dem jeweiligen Ohrläppchen liegenden Bereich hin erstrecken, zur Führung von unteren Bandabschnitten der Kopfbandanordnung. Dadurch wird es auf vorteilhafte Weise möglich, den Verlauf der unteren Kopfbandabschnitte über den Wangenbereich des Anwenders vorteilhaft auf die individuelle Kopfform abzustimmen.

Gemäß einem weiteren Aspekt der vorliegenden Erfindung ist die Stützstruktur vorzugsweise derart ausgebildet, dass diese einen oberen Randabschnitt mit zwei oberen Schenkeln aufweist, die sich von einer, im Nackenbereich liegenden Ausgangszone ausgehend, in einer über den Ohrenbereich weisenden Ausrichtung erstrecken.

Weiterhin wird eine Kopfbandeinrichtung zur Applikation einer Atemmaske an einem Anwender beschrieben, mit einem flexiblen Bandkorpus, der obere und untere Bandabschnitte aufweist, zur Übertragung der zur Applikation der Atemmaske erforderlichen Maskenhaltekräfte, wobei der Bandkorpus zumindest abschnittsweise aus einem geschäumten Kunststoffmaterial gefertigt ist.

Dadurch wird es auf vorteilhafte Weise möglich, eine sich durch Polstereigenschaften auszeichnende Kopfbandeinrichtung ohne erhebliche Zuschnittsveriuste als Serienteil zu fertigen.

Gemäß einem Aspekt wird das zur Bildung des Bandkorpus vorgesehene Kunststoffmaterial durch Einspritzen des Kunststoffmaterials in einen entsprechenden Formraum zu dem Bandkorpus geformt. Der Bandkorpus ist in vorteilhafter Weise derart ausgebildet, dass dieser im Bereich seiner Außenflächen verhautet ist Der Verhautungseffekt kann erreicht werden, indem der entsprechende Forminnenraum mit einer Beschichtung versehen wird. Die Beschichtung kann insbesondere durch einen Pulverbeschichtungsvorgang oder durch Einlegefolien erreicht werden.

Gemäß einem weiteren Aspekt ist der Bandkorpus mit einer zugsteifen Einlage versehen. Diese zugsteife Einlage ist vorzugsweise aus einem thermoplastischen Kunststoffmaterial, insbesondere einem Nylon- oder Polyamidmaterial, gefertigt. Die in den Bandkorpus eingebundene Einlage kann zumindest abschnittsweise derart ausgebildet sein, dass diese abschnittsweise eine formstabile Einlage bildet. Die Polstereigenschaften, insbesondere die Polsterdicke des Bandkorpus, kann derart variieren, dass insbesondere in Bandzonen mit erhöhter Flächenpressung dickere Polsterstärken vorherrschen.

Gemäß einem anderen Aspekt ist der Bandkorpus mit Schließmitteln versehen zur Ankoppelung eines Bandlaschenabschnitts oder Maskenbefestigungselementen in einstellbarer Weise. Dadurch wird es möglich, über die Kopfbandeinrichtung den Maskenanpressdruck präzise einzustellen. Die Schließmittel umfassen vorzugsweise eine Arretierstruktur, die integral mit der Kopfbandeinrichtung ausgebildet ist. Die Arretierstruktur kann derart ausgebildet sein, dass ein Arretiereffekt auf Grundlage einer Kraft und/oder formschlüssigen Koppelung mit einer entsprechenden Gegenstruktur erreicht werden kann. Es ist möglich, die Arretierstruktur so auszubilden, dass diese insbesondere integral mit der zugkraftaufnehmenden bzw. zugsteifen Einlage ausgebildet ist. Die zugsteife Einlage kann derart ausgebildet sein, dass diese ein bestimmtes Biegeverhalten aufweist. Dieses bestimmte Biegeverhalten kann insbesondere durch die Ausbildung von Aussparungen in der zugsteifen Einlage sowie durch Abstimmung der Dicke der zugsteifen Einlage erreicht werden.

Gemäß einem weiteren Aspekt umfassen die Schließmittel eine Schlitteneinrichtung, die an der Kopfbandeinrichtung in unterschiedlichen Schließstellungen arretierbar ist. Die Schlitteneinrichtung kann hierbei einen Arretiermechanismus umfassen, zur Arretierung der Schlitteneinrichtung an der Arretierstruktur.

Weiterhin wird ein Verfahren zur Herstellung einer Kopfbandeinrichtung beschrieben, bei welchem im Rahmen eines ersten Kunststoffspritzschrittes zur Bildung einer zugsteifen Einlage ein entsprechendes Kunststoffmaterial in einen zur Bildung der Einlage konfigurierten Formwerkzeugraum eingespritzt wird und im Rahmen eines nachfolgenden Kunststoffspritzschrittes ein porenbildendes Kunststoffmaterial in einen erweiterten Formraum derart eingespritzt wird, dass das porenbildende Material unter Bildung eines Polsterabschnitts die zugsteife Einlage zumindest abschnittsweise ummantelt.

Die folgenden Aspekte sind auch offenbart:
1. Kopfbandeinrichtung zur Applikation einer Atemmaske an einem Anwender mit einem flexiblen Bandkorpus, der obere und untere Bandabschnitte (3, 4) aufweist, zur Übertragung der zur Applikation der Atemmaske erforderlichen Maskenhaltekräfte, wobei der Bandkorpus zumindest abschnittsweise aus einem geschäumten Kunststoffmaterial gefertigt ist.
2. Kopfbandeinrichtung nach Aspekt 1, wobei der Bandkorpus durch Einspritzen eines porenbildenden Kunststoffmaterials in einen Werkzeug-Formraum gebildet ist.
3. Kopfbandeinrichtung nach Aspekt 1 oder 2, wobei das Kunststoffmaterial im Bereich seiner Außenfläche verhautet ist.
4. Kopfbandeinrichtung nach wenigstens einem der Aspekte 1 bis 3, wobei der Bandkorpus, mit einer zugsteifen Einlage versehen ist.
5. Kopfbandeinrichtung nach wenigstens einem der Aspekte 1 bis 4, wobei der Bandkorpus mit einer formstabilen Einlage versehen ist.
6. Kopfbandeinrichtung nach wenigstens einem der Aspekte 1 bis 5, wobei der Bandkorpus mit Schließmitteln versehen ist, zur Ankoppelung eines Bandlaschenabschnitts in einstellbar veränderbarer Weise.
7. Kopfbandeinrichtung nach wenigstens einem der Aspekte 1 bis 6, wobei die Schließmittel eine Arretierstruktur umfassen, die integral mit der Kopfbandeinrichtung ausgebildet ist.
8. Kopfbandeinrichtung nach wenigstens einem der Aspekte 1 bis 7, wobei die Schließmittel eine Schlitteneinrichtung umfassen, die an der Kopfbandeinrichtung in unterschiedliche Schließstellungen bringbar ist.
9. Kopfbandeinrichtung nach wenigstens einem der Aspekte 1 bis 8, wobei das die Schlitteneinrichtung einen Arretiermechanismus umfasst, zur Arretierung der Schlitteneinrichtung an der Arretierstruktur.
10. Verfahren zur Herstellung einer Kopfbandeinrichtung, bei welchem im Rahmen eines ersten Kunststoffspritzschritts ein zur Bildung einer zugsteifen Einlage vorgesehenes Kunststoffmaterial in einen Formwerkzeugraum eingespritzt wird und im Rahmen eines nachfolgenden Kunststoffspritzschritts ein porenbildendes Kunststoffmaterial in ein Formwerkzeug derart eingebracht wird, dass dieses die zugsteife Einlage unter Bildung eines Polsterabschnitts zumindest abschnittsweise ummantelt.
11. Applikationsvorrichtung für eine Atemmaske mit, einer Kopfbandeinrichtung, die sich in Applikationsposition um den Hinterkopfbereich eines Anwenders erstreckt, wobei die Kopfbandeinrichtung mit einer Stützstruktur ausgestattet ist und die Stützstruktur aus einem Material gefertigt ist, das wenigstens einmal temporär in einen Zustand bringbar ist, in welchem die Kopfbandanordnung zumindest abschnittsweise an die Hinterkopftektur des Anwenders individuell anpassbar ist.
12. Applikationsvorrichtung nach Aspekt 11, wobei die Stützstruktur aus einem nach Erwärmung auf eine Temperatur über 300C plastisch verformbaren Material gefertigt ist.
13. Applikationsvorrichtung nach Aspekt 11 oder 12, wobei die Stützstruktur aus einem thermo-plastischen Kunststoffmaterial gefertigt ist.
14. Applikationsvorrichtung nach wenigstens einem der Aspekte 11 bis 13, wobei die Stützstruktur eine Versteifungslage bildet.
15. Applikationsvorrichtung nach wenigstens einem der Aspekte 11 bis 14, wobei die Stützstruktur einen, der Hinterkopfwölbung angepassten Verlauf aufweist.
16. Applikationsvorrichtung nach wenigstens einem der Aspekte 11 bis 15, wobei die Stützstruktur auf ihrer in Applikationsposition dem Anwender zugewandten Innenseite, mit einer Polsterung versehen ist.
17. Applikationsvorrichtung nach wenigstens einem der Aspekte 11 bis 16, wobei die Stützstruktur mit Durchbrechungen versehen ist.
18. Applikationsvorrichtung nach wenigstens einem der Aspekte 11 bis 17, wobei die Stützstruktur lösbar mit der Kopfbandeinrichtung gekoppelt ist.
19. Applikationsvorrichtung nach wenigstens einem der Aspekte 11 bis 18, wobei die Stützstruktur Armabschnitte aufweist, die sich von einem Hauptflächenabschnitt ausgehend in Richtung der Kopfbandabschnitte erstrecken.
20. Applikationsvorrichtung nach wenigstens einem der Aspekte 11 bis 19, wobei die Stützstruktur einen unteren Randbereich mit zwei unteren Schenkeln (S1, S2) aufweist, die sich in Applikationsposition zum Wangenknochen des Anwenders hin erstrecken, zur Führung von unteren Bandabschnitten (9) der Kopfbandanordnung.
21. Applikationsvorrichtung nach wenigstens einem der Aspekte 11 bis 20, wobei die Stützstruktur einen oberen Randabschnitt mit zwei oberen Schenkeln (S3, S4) aufweist, die sich von einer im Nackenbereich liegenden Ausgangszone ausgehend in einer über den Ohrenbereich weisenden Ausrichtung erstrecken.

Weitere Einzelheiten und Merkmale der Erfindung ergeben sich aus der nachfolgenden Beschreibung in Verbindung mit der Zeichnung. Es zeigt:
- **Figur 1a**: eine Seitenansicht eines Anwenders mit einer durch die erfindungsgemäße Kopfbandeinrichtung applizierten Atemmaske,
- **Figur 1b**: eine Skizze zur Erläuterung einer erfindungsgemäßen Kopfbandeinrichtung,
- **Figur 2a**: eine Querschnittansicht eines Bandkorpus ohne zugsteife Einlage,
- **Figur 3**: eine Bandkorpus-Querschnittskizze zur Erläuterung einer Variante mit zugsteifer Einlage,
- **Figur 4**: eine Skizze zur Erläuterung eines an einem erfindungsgemäßen Kopfband ausgebildeten Arretierabschnitts,
- **Figur 5a**: eine Schnittansicht zur Erläuterung des Querschnittprofils eines Bandkorpus mit integral mit diesem ausgebildeter Arretiereinrichtung,
- **Figur 5b**: eine, der strukturgemäß Figur 5a, zugeordnete Schlitteneinrichtung,
- **Figur 6a**: eine weitere Querschnittsvariante eines Bandkorpus mit darin ausgebildeter zugsteifer Einlage sowie integral mit der zugsteifen Einlage ausgebildeter Arretiereinrichtung,
- **Figur 6b**: eine Skizze zur Erläuterung eines der Arretiereinrichtung nach Figur 6a zugeordneten Schlitteneinrichtung,
- **Figur 7**: eine perspektivische Skizze zur Erläuterung weiterer Einzelheiten eines aus einem Schaummaterial gefertigten Kopfbands für eine Atemmaske mit einer zugsteifen Einlage und einer integral mit dieser ausgebildeten Arretiereinrichtung,
- **Figur 8**: eine Skizze zur weiteren Funktionsbeschreibung der Kopfbandeinrichtung gemäß Figur 7.
- **Figur 9**: eine Seitenansicht eines Atemmaskenanwenders mit einer, an diesem über eine Kopfbandanordnung applizierten Atemmaske,
- **Figur 10**: eine Ansicht des Hinterkopfbereichs eines Maskenanwenders zur Erläuterung einer bevorzugten Gestaltung der Kopfbandanordnung in diesem Bereich,
- **Figur 11**: eine Skizze zur Erläuterung des Aufbaus einer erfindungsgemäßen, von einer Atemmaskenanordnung getrennten, Applikationsvorrichtung,
- **Figur 12**: eine Skizze zur Erläuterung einer bevorzugten Querschnittsgestaltung der Applikationsvorrichtung im Bereich der Stützstruktur.

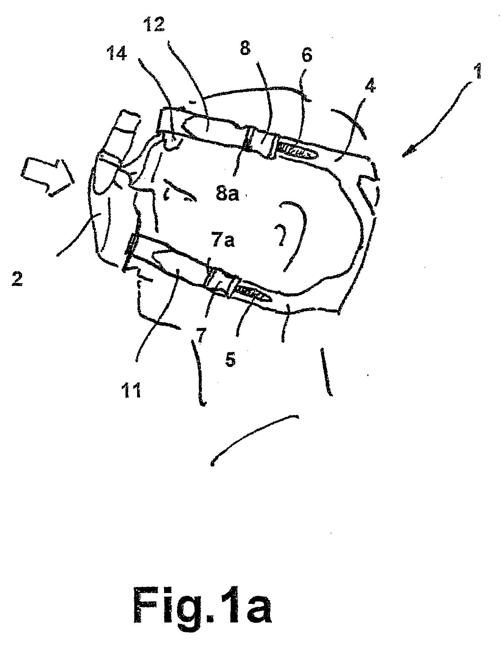

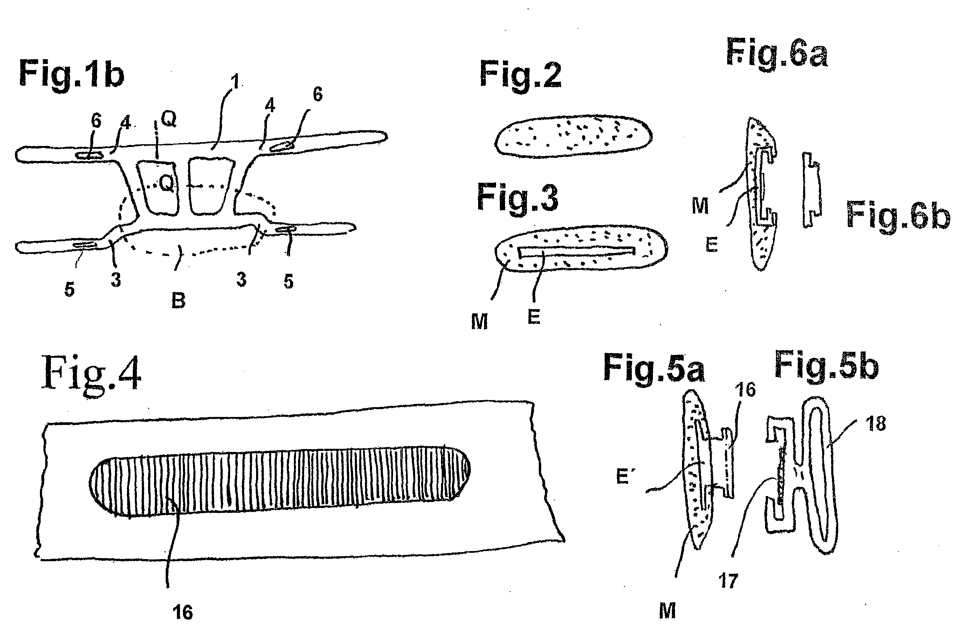

Der in Figur 1 dargestellte Atemmaskenanwender trägt eine, durch eine erfindungsgemäße Kopfbandeinrichtung 1 nasal applizierte Atemmaske 2. Die Fixierung der Atemmaske 2 erfolgt über untere Kopfbandabschnitte 3 und obere Kopfbandabschnitte 4. Die Kopfbandeinrichtung 1 ist aus einem geschäumten Kunststoffmaterial gefertigt, indem dieses im Rahmen eines Kunststoffspritzschrittes in einen entsprechenden Formraumabschnitt eingespritzt wurde.

Die Kopfbandeinrichtung 1 ist mit Arretiereinrichtungen 5, 6 versehen, durch welche Fixiermittel 7, 8 in einstellbar veränderbarer Weise an dem jeweiligen Kopfbandabschnitt 3, 4 festlegbar sind. Die Fixiermittel 7, 8 weisen jeweils Laschenabschnitte 7a, 8a auf, wobei durch diese Laschenabschnitte 7a, 8a weitere, zur Atemmaske 2 vordringende Bandabschnitte 11, 12 hindurchgeführt sind.

Die Kopfbandeinrichtung 1 ist derart ausgebildet, dass der untere Kopfbandabschnitt 3 sich aus dem Nackenbereich, unter den Ohren über den Wangenbereich hin zur Atemmaske erstreckt. Der obere Kopfbandabschnitt 4 erstreckt sich aus dem oberen Hinterkopfbereich in den Stirnbereich des Maskenanwenders. Durch den unteren Kopfbandabschnitt 3 und den oberen Kopfbandabschnitt 4 werden die zur Applikation der Atemmaske 2 erforderlichen Haltekräfte aufgebracht.

Durch den unteren Kopfbandabschnitt 3 wird imwesentlichen der Anpressdruck der Atemmaske 2 auf den Umgebungsbereich der Nase des Maskenanwenders bestimmt. Durch die im oberen Kopfbandabschnitt 4 herrschende Zugkraft wird im Wesentlichen der Anpressdruck einer Stirnauflageeinrichtung 14 auf die Stirn des Maskenanwenders bestimmt.

In Figur 1b ist skizzenhaft eine, in eine Ebene ausgebreitete Kopfbandeinrichtung 1 gezeigt. Diese Kopfbandeinrichtung umfasst die oberen Kopfbandabschnitte 4 sowie die vorangehend genannten unteren Kopfbandabschnitte 3. Die Kopfbandeinrichtung 1 ist aus einem geschäumten Material gefertigt und mit einer zugsteifen Einlage versehen. Die zugsteife Einlage ist in dem hier durch Punktlinien gekennzeichneten Bereich B derart ausgebildet, dass diese um eine zur Auflagefläche imwesentlichen senkrechte Achse eine besonders hohe Torsionssteifigkeit aufweist. Hierdurch wird es möglich, im Bereich der unteren Kopfbandabschnitte 3 eine Bandkröpfung zu realisieren, durch welche die unteren Kopfbandabschnitte 3 in vorteilhafter Weise unter dem Ohrbereich des Anwenders auf dessen Wangenbereich hingeführt werden können. Die Kopfbandeinrichtung 1 ist, wie angedeutet, durch integral mit dieser ausgebildeten Arretierstrukturen 5, 6 versehen, auf die nachfolgend noch näher eingegangen werden wird.

Figur 2b zeigt eine Ausführungsform der erfindungsgemäßen Kopfbandeinrichtung, die insgesamt aus einem Poren bildenden Kunststoffmaterial gefertigt ist.

Figur 3 zeigt eine Querschnittsskizze Q/Q zur Erläuterung einer Variante des Bandkorpus mit darin vorgesehener zugsteifer Einlage E. Die zugsteife Einlage ist aus einem thermo-plastischen Kunststoffmaterial, beispielsweise Nylon oder Polyamid, gefertigt. Die zugsteife Einlage E ist in ein Schaummaterial M eingebettet. Das Schaummaterial M ist an die zugsteife Einlage E angespritzt.

Figur 4 zeigt eine Skizze zur Erläuterung einer, gemäß einer besonders bevorzugten Ausführungsform der Erfindung, integral mit der zugsteifen Einlage E ausgebildeten Arretierstruktur 16. Die Arretierstruktur 16 ist als querverzahnter Bahnabschnitt ausgebildet und kann insbesondere den in Figur 5a dargestellten Querschnitt aufweisen. Der Bahnkörper der Arretierstruktur 16 ist derart ausgebildet, dass dieser eine Führungseinrichtung für ein Schlittenelement bildet. Die Rastposition des Schlittenelements an der Bahneinrichtung ist in einstellbarer Weise veränderbar.

Figur 5b zeigt skizzenhaft eine mögliche Variante eines Schlittenelements, wie dies auf die Bahneinrichtung gemäß Figur 5a bzw. gemäß Figur 4 aufsetzbar ist. Das Schlittenelement umfasst einen hier nicht näher dargestellten Arretiermechanismus 17, der mit der Arretierstruktur 16 selektiv in Eingriff bringbar ist. An dem Schlittenelement ist ein Laschenabschnitt 18 ausgebildet zur Aufnahme eines Bandabschnitts 11, 12 wie in Figur 1 a dargestellt.

Figur 6a zeigt eine weitere Querschnittsvariante der erfindungsgemäßen Kopfbandeinrichtung im Bereich der Arretiereinrichtung. Der Kopfbandkorpus umfasst auch hier eine, aus einem geschäumten Material gefertigte, Polsterlage M sowie eine darin eingebettete zugsteife Einlage E. Die zugsteife Einlage bildet auch hier Teil einer Arretiereinrichtung, die abweichend von der Variante nach Figur 5a weitgehend versenkt im Bandkorpus aufgenommen ist. In die, in den Bandkorpus hineinversenkte Bahneinrichtung ist ein Schlittenelement einsetzbar, das über einen, hier ebenfalls nicht näher dargestellten Arretiermechanismus, in einstellbar veränderbarer Weise an der entsprechende Führung festlegbar ist.

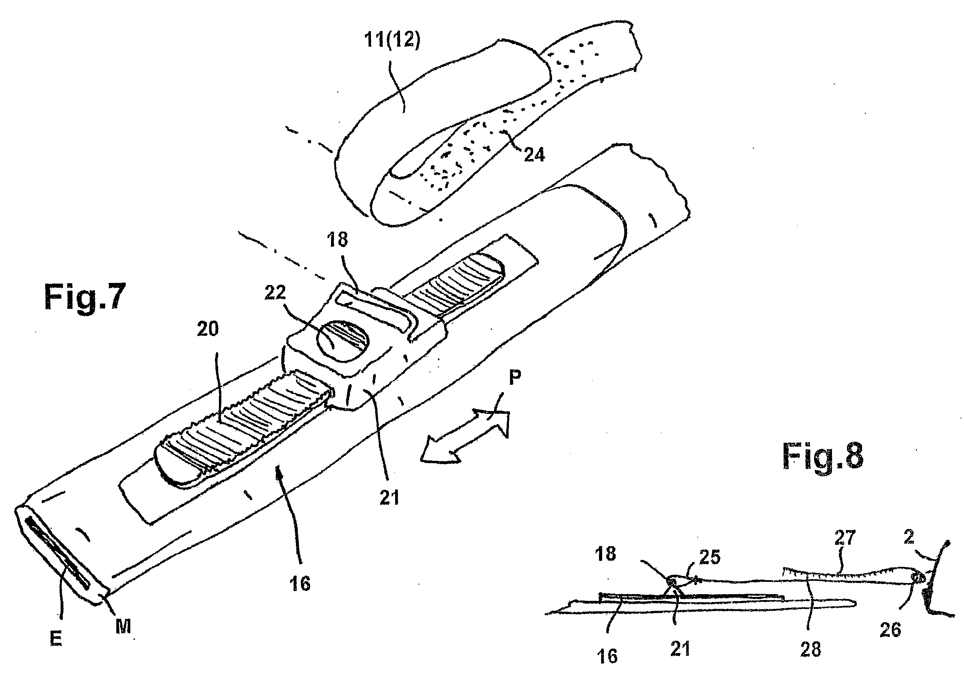

Figur 7 zeigt eine Kopfbandvariante mit einem, aus einem geschäumten Material gebildeten, Polsterkörper M und einer darin eingebetteten zugsteifen Lage E. Die zugsteife Lage E trägt eine Arretiereinrichtung 16, die eine mit Rastzähnen 20 versehene Führungsbahn bildet. Auf dieser Führungsbahn ist ein Schlittenelement 21 angeordnet. Das Schlittenelement 21 kann unter Betätigung eines Druckknopfs 22 und hierdurch verursachte Entriegelung des nicht näher dargestellten Arretiermechanismus in die durch das Pfeilsymbol P angedeuteten Richtungen verschoben werden. Das Schlittenelement 21 trägt einen Laschenabschnitt 18, durch welchen ein Bandabschnitt 11,12 der Kopfbandeinrichtung hindurchführbar ist. Der Bandabschnitt 11, 12 ist mit einer Klettverschlusseinrichtung 24 versehen, durch welche eine grobe Voreinstellung der effektiven Länge des jeweiligen Kopfbandabschnitts 3, 4 bewerkstelligbar ist.

Alternativ zu der hier gezeigten Anbindung des Kopfbandabschnitts 11, 12 an den Laschenabschnitt 18 ist es auch möglich, das Schlittenelement 21, wie in Figur 8 schematisch dargestellt, mit einer Atemmaske 2 zu koppeln und zwar indem der Laschenabschnitt 18 in eine fest vernähte Bandlasche 25 eingebettet ist und die Ankoppelung an die Atemmaske 2 über eine, durch einen Haltebügel 26 der Atemmaske 2 hindurchgeführte Bandlasche 27 bewerkstelligt ist, die wiederum durch eine Klettverschlusseinrichtung 28 lösbar ausgebildet ist. Das Schlittenelement 21 ist entlang der Arretiereinrichtung 16 verschiebbar. Die Arretiereinrichtung 16 kann auch durch eine im Bandkorpus versenkt ausgebildete Bahneinrichtung (Figur 6a) verwirklicht sein.

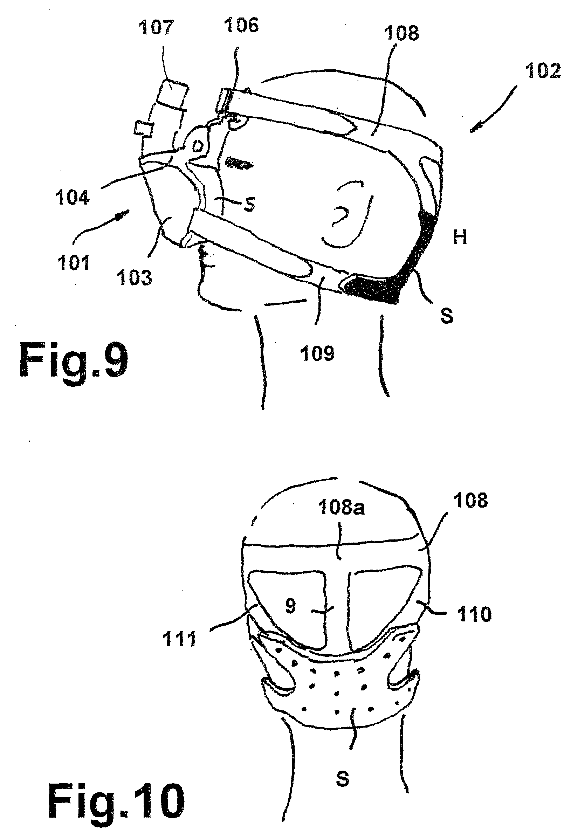

In Figur 9 ist ein Atemmaskenanwender gezeigt, an welchem eine weitere Atemmaskenanordnung 101 über eine, hier als Kopfbandanordnung ausgeführte, Applikationsvorrichtung 102 fixiert ist. Die Atemmaskenanordnung 101 umfasst einen Maskenbasiskörper 103, der über eine Dichtlippe 105 abdichtend auf dem Nasenbereich des Anwenders aufsitzt. Der Maskenbasiskörper 103 ist über eine Rahmenstruktur und eine mit dieser gekoppelte Stirnauflageeinrichtung 106 am Anwender fixiert. Die Zufuhr des Atemgases zu dem, durch den Maskenbasiskörper 103 definierten Maskeninnenraum, kann über die hier nur skizzenhaft dargestellte Anschlusseinrichtung 107 erfolgen.

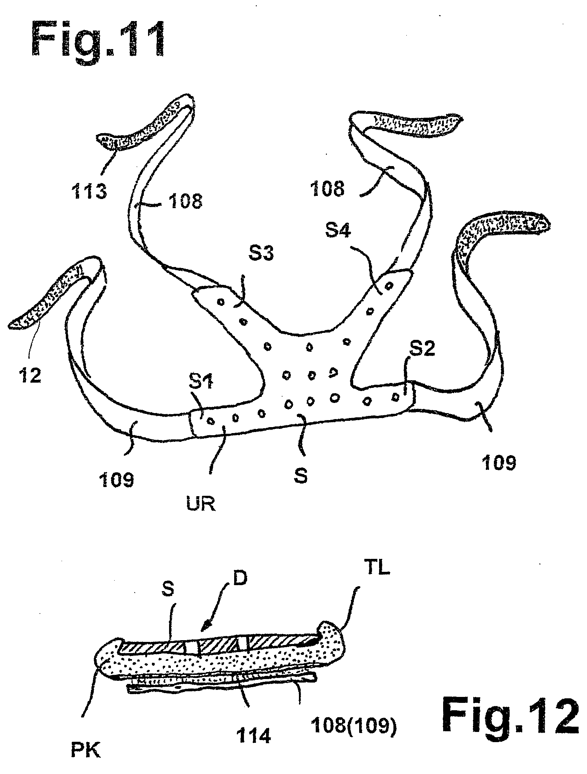

Die Applikationsvorrichtung 102 umfasst einen oberen Kopfbandabschnitt 108, der sich vom Hinterkopfbereich H ausgehend in den Bereich der Stirnauflageeinrichtung 106 erstreckt. Weiterhin umfasst die Applikationsvorrichtung auch einen unteren Kopfbandabschnitt 109, der sich vom Nackenbereich des Anwenders ausgehend über dessen Wangenknochen in den Bereich des Maskenbasiskörpers 103 erstreckt. Die Koppelung des jeweiligen Kopfbandabschnitts 108, 109 mit der Atemmaskenanordnung 101 ist bei dieser Ausführungsform über Klettverschlussstrukturen bewerkstelligt, wie diese nachfolgend noch in Verbindung mit Figur 11 beschrieben werden. Die Applikationsvorrichtung ist mit einer Stützstruktur S versehen, die bei diesem Ausführungsbeispiel durch ein formsteifes Plattenmaterial gebildet ist. Das formsteife Plattenmaterial ist aus einem Werkstoff gefertigt, der unter bestimmten Bedingungen plastisch verformbar und damit an die individuelle Hinterkopftektur des Anwenders anpassbar ist. Bei diesem Ausführungsbeispiel wird die Anpassbarkeit erreicht, indem das zur Bildung der Stützstruktur vorgesehene Material ein thermo-verformbares Kunststoffmaterial ist. Die Materialeigenschaften dieses Kunststoffmaterials sind derart abgestimmt, dass dieses bei Temperaturen im Bereich ab ca. 50° Celsius plastisch verformbar ist. Durch die Verwendung der erfindungsgemäß an die individuelle Hinterkopftektur angepassten Stützstruktur wird es möglich, die zur Applikation der Atemmaskenanordnung 101 erforderlichen Haltekräfte in einer, unter physiologischen Gesichtspunkten, vorteilhaften Weise aufzubringen. Durch die Anpassung der Stützstruktur S an die individuelle Kopfform des Anwenders wird es insbesondere möglich, den Verlauf der unteren Kopfbandanordnung 109 vorteilhaft festzulegen und zudem eine hohe Rutsch- und Verdrehsicherheit zu erreichen.

Figur 10 zeigt eine Ansicht der vorangehend beschriebenen Applikationsvorrichtung von hinten. Wie aus dieser Ansicht ersichtlich, ist der obere Kopfbandabschnitt 8 über den Hinterkopfbereich des Anwenders geführt. Der den Hinterkopfbereich überbrückende Abschnitt 8a des oberen Kopfbandabschnitts ist durch einen senkrechten Steg 9 sowie durch Diagonalstege 110, 111 mit der im Hinterkopfbereich des Anwenders verlaufenden unteren Bandanordnung der Applikationsvorrichtung 102 gekoppelt. Die Kopfbandeinrichtung ist im Hinterkopfbereich des Patienten mit jener Stützstruktur S ausgestattet. Diese Stützstruktur S ist an die individuelle Hinterkopfform des Anwenders angepasst. Die Stützstruktur S ist mit Durchbrechungen versehen, um hierdurch eine erhöhte Dampfdurchlässigkeit zu erreichen.

Wie aus Figur 11 ersichtlich, umfasst die Stützstruktur S einen unteren Randabschnitt UR, der Schenkelabschnitte S1, S2 aufweist, die derart angeordnet sind, dass diese den ihnen jeweils zugeordneten Bandabschnitt 109 derart führen, dass sich dieser Bandabschnitt in vorteilhafter Weise über den Wangenbereich des Maskenanwenders erstreckt. Die Stützstruktur weist bei diesem Ausführungsbeispiel zwei weitere Schenkelabschnitte S3, S4 auf, die dazu dienen, die oberen Bandabschnitten 108 der Kopfbandeinrichtung zu führen.

Die Kopfbandabschnitte 108, 109 sind mit Klettverschlusseinrichtungen 112, 113 versehen, die dazu dienen, die Kopfbandabschnitte 8, 9 lösbar mit entsprechenden Laschenabschnitten der Atemmaskenanordnung 101 zu koppeln.

In Figur 12 ist in Form einer vereinfachten Querschnittsskizze eine bevorzugte Ausführungsform der Applikationsvorrichtung im Bereich der Stützstruktur S dargestellt. Die Stützstruktur S ist mit einem Poisterkörper PK versehen. Der Polsterkörper PK ist bei diesem Ausführungsbeispiel aus einem Schaumstoffmaterial gebildet, das in eine textile Lage TL eingebettet ist. Die textile Lage TL kann derart ausgebildet sein, dass diese eine Koppelungsfläche 114 bereitstellt, die es ermöglicht, die derart ausgebildete, gepolsterte Stützstruktureinrichtung lösbar über eine Klettverschlusseinbindung mit der Kopfbandeinrichtung 108, 109 zu koppeln. Die Stützstruktur S ist mit Durchbrechungen D versehen, zur Erhöhung der Dampfdurchlässigkeit der Stützstruktur S.

Alternativ zu der Bewerkstelligung der Stützstruktur S durch ein thermo-verformbares Material ist es auch möglich, die Stützstruktur aus einem Material zu fertigen, das in anderer Weise an die Hinterkopfform des Anwenders anpassbar ist und diese individuelle Form hinreichend formstabil beibehält. Dies kann insbesondere erreicht werden durch Verwendung aushärtender Werkstoffe, wie beispielsweise Harze. Die Stützstruktur S kann derart vorgeformt sein, dass diese einen Krümmungsverlauf aufweist, der im Grunde der wahrscheinlichsten Hinterkopfgestalt Rechnung trägt, so dass eine weitere Anpassung ggf. nicht erforderlich ist.

Das Konzept der Verwendung einer temporär plastifizierbaren formsteifen Struktur kann in vorteilhafter Weise in Kombination mit dem Konzept der Bildung des Bandkorpus durch Schaum-Spritzformen Anwendung finden. Insbesondere ist es möglich, die hinsichtlich des Schaum-Spritzformkonzeptes genannten zugsteifen Strukturen zumindest im Hinterkopfbereich aus einem temporär, z.B. unter Wärmeeinwirkung plastifizierbaren oder zumindest hinreichend anformbaren Material zu fertigen.

## Patentansprüche

1. Applikationsvorrichtung (102) für eine Atemmaske mit einer Kopfbandeinrichtung (1) mit oberen und unteren Kopfbandabschnitten (108,109), die sich in Applikationsposition um den Hinterkopfbereich eines Anwenders erstreckt, wobei sich die oberen und unteren Kopfbandabschnitte vom Hinterkopfbereich eines Anwenders zur Atemmaske hin erstrecken, wobei sich der obere Kopfbandabschnitt (108) zum Stirnbereich hin erstreckt und der untere Kopfbandabschnitt (109) sich über die Wangenknochen erstreckt, wobei die Kopfbandeinrichtung mit einer vorgeformten Stützstruktur (S) ausgestattet ist und die vorgeformte Stützstruktur (S) einen Krümmungsverlauf aufweist, der an die Hinterkopfwölbung angepasst ist, **dadurch gekennzeichnet**
**daß** die Stützstruktur aus einem Material gefertigt ist, das wenigstens einmal temporär in einen Zustand bringbar ist, in welchem die Kopfbandeinrichtung zumindest abschnittsweise an die Hinterkopftektur des Anwenders individuell anpassbar ist, und wobei die Stützstruktur aus einem thermoplastischen Kunststoffmaterial gefertigt ist.

2. Applikationsvorrichtung (102) nach Anspruch 1, **dadurch gekennzeichnet, dass** die Stützstruktur (S) aus einem nach Erwärmung auf eine Temperatur über 30°C, weiter bevorzugt über 50°C oder über 300°C, plastisch verformbaren Material gefertigt ist.

3. Applikationsvorrichtung (102) nach Anspruch 1 oder 2, **dadurch gekennzeichnet, dass** die Stützstruktur (S) durch ein formsteifes Plattenmaterial gebildet ist.

4. Applikationsvorrichtung (102) nach wenigstens einem der Ansprüche 1 bis 3, **dadurch gekennzeichnet, dass** die Stützstruktur (S) eine Versteifungslage bildet.

5. Applikationsvorrichtung (102) nach wenigstens einem der Ansprüche 1 bis 4, **dadurch gekennzeichnet, dass** die Stützstruktur (S) auf ihrer in Applikationsposition dem Anwender zugewandten Innenseite mit einer Polsterung versehen ist.

6. Applikationsvorrichtung (102) nach wenigstens einem der Ansprüche 1 bis 5, **dadurch gekennzeichnet, dass** die Stützstruktur (S) mit Durchbrechungen (D) versehen ist.

7. Applikationsvorrichtung (102) nach wenigstens einem der Ansprüche 1 bis 6, **dadurch gekennzeichnet, dass** die Stützstruktur (S) lösbar mit der Kopfbandeinrichtung (1) gekoppelt ist.

8. Applikationsvorrichtung (102) nach wenigstens einem der Ansprüche 1 bis 7, **dadurch gekennzeichnet, dass** die Stützstruktur (S) Armabschnitte aufweist, die sich von einem Hauptflächenabschnitt ausgehend in Richtung der Kopfbandabschnitte (108,109) erstrecken.

9. Applikationsvorrichtung (102) nach wenigstens einem der Ansprüche 1 bis 8, **dadurch gekennzeichnet, dass** die Stützstruktur (S) einen unteren Randbereich mit zwei unteren Schenkeln (S1, S2) aufweist, die sich in Applikationsposition zum Wangenknochen des Anwenders hin erstrecken, zur Führung von unteren Kopfbandabschnitten (109) der Kopfbandeinrichtung (1).

10. Applikationsvorrichtung (102) nach wenigstens einem der Ansprüche 1 bis 9, **dadurch gekennzeichnet, dass** die Stützstruktur (S) einen oberen Randabschnitt mit zwei oberen Schenkeln (S3, S4) aufweist, die sich von einer im Nackenbereich liegenden Ausgangszone ausgehend in einer über den Ohrenbereich weisenden Ausrichtung erstrecken.

11. Applikationsvorrichtung (102) nach wenigstens einem der Ansprüche 1 bis 10, **dadurch gekennzeichnet, dass** die Kopfbandeinrichtung (1) ein geschäumtes Material umfasst.

12. Applikationsvorrichtung (102) nach wenigstens einem der vorhergehenden Ansprüche, wobei die Stützstruktur (S) formstabil ist.

## Claims

1. An application device (102) for a breathing mask comprising a headband means (1) with upper and lower headband portions (108, 109), wherein, in the application position, said headband means (1) extends around the area of the back of a user's head, wherein the upper and lower headband portions extend from the back of a user's head to the breathing mask, wherein the upper headband portion (108) extends to the forehead area and the lower headband portion (109) extends across the cheek area, wherein the headband means is provided with a preformed supporting structure (S) and the course of the curvature of said preformed supporting structure (S) is adapted to the curvature of the back of a user's head, **characterized in that** the supporting structure is made of a material which at least once can be brought temporarily in a state in which at least portions of the headband means can be adapted individually to the shape of the back of the user's head, and wherein the supporting structure is made of a thermoplastic material

2. The application device (102) according to claim 1, **characterized in that** the supporting structure (S) is made of a material which is plastically deformable after having been heated to a temperature of above 30°C, more preferably above 50°C or above 300°C.

3. The application device (102) according to claim 1 or 2, **characterized in that** the supporting structure (S) is formed of an inherently stable plate material.

4. The application device (102) according to at least one of claims 1 to 3, **characterized in that** the supporting structure (S) is a stiffening layer.

5. The application device (102) according to at least one of claims 1 to 4, **characterized in that** the supporting structure (S) is provided with a cushion on its inner surface which faces the user in the application position.

6. The application device (102) according to at least one of claims 1 to 5, **characterized in that** the supporting structure (S) is provided with openings (D).

7. The application device (102) according to at least one of claims 1 to 6, **characterized in that** the supporting structure (S) is detachably coupled to the headband means (1).

8. The application device (102) according to at least one of claims 1 to 7, **characterized in that** the supporting structure (S) comprises arm portions which extend from a main surface portion in the direction of the headband portions (108, 109).

9. The application device (102) according to at least one of claims 1 to 8, **characterized in that** the supporting structure (S) comprises a lower edge portion having two lower legs (S1, S2), which, in the application position, extend towards the cheekbone of the user for guiding lower band portions (109) of the headband means (1).

10. The application device (102) according to at least one of claims 1 to 9, **characterized in that** the supporting structure (S) comprises an upper edge portion having two upper legs (S3, S4), which extend from a starting zone in the neck area in a direction extending across the ear area.

11. The application device (102) according to at least one of claims 1 to 10, **characterized in that** the headband means (1) comprises a foamed material.

12. The application device (102) according to at least one of the preceding claims, wherein the supporting structure (S) is dimensionally stable.

## Revendications

1. Dispositif d'application (102) pour un masque respiratoire avec un dispositif de bandeau serre-tête (1) avec des segments de bandeau serre-tête supérieurs et inférieurs (108, 109), le dispositif de bandau serre-tête, dans la position d'application, s'étendant autour de la partie arrière de la tête d'un utilisateur, dans lequel les segments de bandeau serre-tête supérieurs et inférieurs s'étendent de la partie arrière de la tête d'un utilisateur jusqu'au masque respiratoire, dans lequel le segment de bandeau serre-tête supérieur (108) s'étend vers la partie frontale et le segment de bandeau serre-tête inférieur (109) s'étend à travers les os de joue, le dispositif de bandeau serre-tête étant aménagé avec une structure de support préformée (S) et la structure de support préformeée (S) présente un gradient de courbure adapté à la courbure de l'arrière de la tête, charactérisé en ce que la structure de support est fabriquée d'un matériau qui peut être mis, au moins une fois et temporairement, dans un état où le dispositif de bandeau serre-tête est au moins partiellement adaptable individuellment à la forme de l'arrière-tête de l'utilisateur, et dans lequel la structure de support est fabriquée d'un matériau thermoplastique.

2. Dispositif d'application (102) selon la revendication 1, charactérisé en ce que la structure de support (S) est fabriquée d'un matériau deformable plastiquement après chauffage à une température supérieure à 30°C, plus préféré supérieure à 50°C ou 300°C.

3. Dispositif d'application (102) selon la revendication 1 ou 2, charactérisé en ce que la structure de support (S) est formée d'un matériau en plaques rigide.

4. Dispositif d'application (102) selon au moins une des revendications 1 à 3, charactérisé en ce que la structure de support (S) forme une couche de rigidité.

5. Dispositif d'application (102) selon au moins une des revendications 1 à 4, charactérisé en ce que la structure de support (S) est pourvue d'un rembourrage à son intérieur orienté vers le visage de l'utilisateur dans la position d'application.

6. Dispositif d'application (102) selon au moins une des revendications 1 à 5, charactérisé en ce que la structure de support (S) est pourvue d'ouvertures (D).

7. Dispositif d'application (102) selon au moins une des revendications 1 à 6, charactérisé en ce que la structure de support (S) est couplée au dispositif de bandeau serre-tête (1) de manière détachable.

8. Dispositif d'application (102) selon au moins une des revendications 1 à 7, charactérisé en ce que la structure de support (S) comprend des parties de bras s'étendant d'une partie de surface principale dans la direction des segments de bandeau serre-tête (108, 109).

9. Dispositif d'application (102) selon au moins une des revendications 1 à 8, charactérisé en ce que la structure de support (S) comprend une partie de bord inférieure avec deux cuisses inférieures (S1, S2) s'étendant, dans la position d'application, vers l'os de joue de l'utilisateur, pour le guidage de segments de bandeaux serre-tête inférieurs (109) du dispositif de bandeau serre-tête (1).

10. Dispositif d'application (102) selon au moins une des revendications 1 à 9, charactérisé en ce que la structure de support (S) comprend une partie/un segment de bord supérieure avec deux cuisses supérieures (S3, S4) s'étendant d'une zone de départ de la partie de nuque dans une direction à travers la partie d'oreille.

11. Dispositif d'application (102) selon au moins une des revendications 1 à 10, charactérisé en ce que le dispositif de bandeau serre-tête (1) comprend un matériau expansé.

12. Dispositif d'application (102) selon au moins une des revendications précédentes, charactérisé en ce que la structure de support (S) est rigide.
